(19) Europäisches Patentamt
European Patent Office
Office européen des brevets

(11) **EP 3 391 027 B1**

(12) **FASCICULE DE BREVET EUROPEEN**

(45) Date de publication et mention
de la délivrance du brevet:
**07.10.2020 Bulletin 2020/41**

(21) Numéro de dépôt: **16823237.9**

(22) Date de dépôt: **16.12.2016**

(51) Int Cl.:
**G01N 21/01** *(2006.01)*        **G02B 1/11** *(2015.01)*
**G01N 33/543** *(2006.01)*

(86) Numéro de dépôt international:
**PCT/EP2016/081600**

(87) Numéro de publication internationale:
**WO 2017/103222 (22.06.2017 Gazette 2017/25)**

(54) **SUPPORTS AMPLIFICATEURS DE CONTRASTE UTILISANT UN MATERIAU BIDIMENSIONNEL**

KONTRASTVERSTÄRKENDE TRÄGER MIT ZWEIDIMENSIONALEM MATERIAL

CONTRAST-AMPLIFYING CARRIERS USING A TWO-DIMENSIONAL MATERIAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.12.2015 FR 1562668**

(43) Date de publication de la demande:
**24.10.2018 Bulletin 2018/43**

(73) Titulaires:
- **Commissariat à l'Énergie Atomique et aux Énergies Alternatives**
  **75015 Paris (FR)**
- **Centre National de la Recherche Scientifique**
  **75016 Paris (FR)**
- **Université du Mans**
  **72085 Le Mans Cedex 09 (FR)**

(72) Inventeurs:
- **CAMPIDELLI, Stéphane**
  **78690 Saint Remy L'honore (FR)**
- **CORNUT, Renaud**
  **92320 Chatillon (FR)**
- **DERYCKE, Vincent**
  **78180 Montigny-le-bretonneux (FR)**
- **AUSSERRE, Dominique**
  **72370 Soulitre (FR)**
- **AUSSERRE, Manuel**
  **69009 Lyon (FR)**

(74) Mandataire: **Priori, Enrico et al**
**Marks & Clerk France**
**Immeuble "Visium"**
**22, avenue Aristide Briand**
**94117 Arcueil Cedex (FR)**

(56) Documents cités:
WO-A1-2011/004136      WO-A1-2014/193819
WO-A1-2015/055809      WO-A1-2015/055810
CN-U- 203 217 962      US-A1- 2015 301 039

- **SHAN ZHIFA ET AL: "One-step transfer and doping of large area graphene by ultraviolet curing adhesive", CARBON, vol. 84, 1 avril 2015 (2015-04-01), - 1 avril 2015 (2015-04-01), pages 9-13, XP029132036, ISSN: 0008-6223, DOI: 10.1016/J.CARBON.2014.11.043**
- **Dominique Ausserré ET AL: "Absorbing Backside Anti-reflecting Layers for high contrast imaging in fluid cells", , 29 mai 2014 (2014-05-29), XP055146681, Extrait de l'Internet: URL:http://arxiv.org/abs/1405.7672 [extrait le 2016-08-30]**
- **HOANG NGUYEN ET AL: "Surface Plasmon Resonance: A Versatile Technique for Biosensor Applications", SENSORS, vol. 15, no. 5, 5 mai 2015 (2015-05-05), pages 10481-10510, XP055264403, DOI: 10.3390/s150510481**

**Description**

L'invention porte sur des supports amplificateurs de contraste pour l'observation d'un échantillon, ainsi que sur des procédés de fabrication de tels supports. L'invention porte également sur des procédés d'observation d'échantillons et des procédés de détection ou dosage d'espèces chimiques ou biologiques mettant en œuvre de tels supports.

L'invention est susceptible d'être appliquée à différents domaines techniques, tels que la biologie (détection de biomolécules ou microorganismes, observation de cellules isolées ou de cultures cellulaires), les nanotechnologies (visualisation de nano-objets, tels que des nanotubes), la microélectronique, la science des matériaux, etc.

L'utilisation de couches antireflet (ou couches « λ/4 ») pour augmenter le contraste optique d'un objet observé en microscopie optique par réflexion est une technique connue depuis de nombreuses années et très puissante. Soit $I$ l'intensité lumineuse réfléchie par l'objet à observer, déposé sur un support, et $I_s$ celle réfléchie par le support seul ; alors, le contraste avec lequel l'échantillon est observé vaut $C=(I-I_s)/(I+I_s)$. On comprend que la valeur absolue de ce contraste prend sa valeur maximale (égale à 1) quand $I_s=0$, c'est-à-dire quand le support a une réflectivité nulle, ou bien quand l'objet supporté à une réflectivité nulle. Dans le cas le plus simple, la condition $I_s=0$ est satisfaite en utilisant en tant que support un substrat transparent sur lequel est déposée une couche mince, également transparente, dont l'épaisseur et l'indice de réfraction sont choisis de manière opportune. Dans le cas d'une couche antireflet unique, éclairée en incidence normale avec un milieu incident (dont provient l'éclairage), et un milieu émergent (le substrat) transparents et semi-infinis, on obtient les conditions suivantes :

$$n_1{}^2 = n_0 n_3 \qquad\qquad (1a)$$

$$n_1 e_1 = \lambda/4 \qquad\qquad (1b)$$

où $n_1$ est l'indice de réfraction (réel) de la couche, $n_0$ et $n_3$ les indices de réfraction (également réels) des milieux incident et émergent, $e_1$ l'épaisseur de la couche et $\lambda$ la longueur d'onde d'éclairage.

Pour des milieux incident et émergent donnés, l'équation (1a) détermine de manière univoque l'indice de réfraction de la couche antireflet. Malheureusement, cet indice peut ne pas correspondre à un matériau d'usage courant, ou satisfaisant à diverses contraintes liées à l'application spécifiquement considérée. Par exemple, dans le cas d'une interface air-verre - dont l'intérêt pratique est évident - on obtient $n_1 \cong 1,27$, ce qui nécessite l'utilisation de matériaux composites tels que des aérogels.

Afin de surmonter cet inconvénient, il a été proposé récemment d'utiliser des couches antireflets absorbantes. Voir à ce propos la demande internationale WO2015/055809 ainsi que les articles suivants :

- Ausserré D, Abou Khachfe R, Roussille L, Brotons G, Vonna L, et al. (2014) « Anti-Reflecting Absorbing Layers for Electrochemical and Biophotonic Applications » J Nanomed Nanotechnol 5: 214; et
- Ausserré D., Abou Khachfe R, Amra C., Zerrad M. « Absorbing Backside Anti-reflecting Layers for high contrast imaging in fluid cells. » arXiv:1405.7672 [physics.optics].

Il s'agit là d'un véritable changement de paradigme car - dans le cas des couches « λ/4 » conventionnelles - l'augmentation du contraste résulte d'un effet interférentiel qui met en jeu des réflexions multiples aux interfaces milieu incident/couche et couche/milieu émergent ; or, l'absorption de la lumière à l'intérieur de la couche tend à supprimer l'interférence entre ces réflexions multiples : par conséquent, le concept même d'une « couche antireflet absorbante » parait de prime abord contraire à l'intuition.

L'utilisation d'une couche antireflet absorbante est avantageuse car le degré de liberté additionnel associé à la présence d'une partie imaginaire de l'indice permet de relâcher la contrainte pesant sur la valeur de sa partie réelle. Par ailleurs, alors qu'il est difficile de modifier la partie réelle de l'indice de réfraction d'un matériau, il est relativement simple de modifier sa partie imaginaire (par exemple, en introduisant des impuretés absorbantes ou diffusantes, la diffusion « simulant » une absorption).

En outre, un support amplificateur de contraste comprenant une couche antireflet absorbante doit être utilisé dans une configuration « inversée » ou « face arrière », c'est-à-dire avec éclairage et observation à travers le substrat qui a un indice de réfraction supérieur à celui du milieu émergent (le « milieu ambiant »). Cette configuration est particulièrement adaptée lorsque le substrat forme une fenêtre d'observation et la couche antireflet absorbante est mise en contact avec un milieu aqueux (applications chimiques ou biologiques) ou maintenue dans une enceinte sous vide ou à atmosphère contrôlée (applications telles que les procédés de dépôt).

Les couches antireflets absorbantes connues de l'art antérieur présentent néanmoins un certain nombre d'inconvénients.

Premièrement, malgré le degré de liberté supplémentaire offert au concepteur par l'utilisation d'un indice de réfraction complexe, il est difficile de réaliser des couches antireflets absorbantes « idéales », c'est-à-dire satisfaisant aux deux conditions qui assurent une extinction complète de la réflexion.

Deuxièmement les couches antireflets absorbantes métalliques - qui sont particulièrement intéressantes en principe, entre autres car elles permettent d'appliquer un potentiel électrique à l'échantillon à observer - ne sont pas parfaitement lisses à l'échelle atomique. En outre, lorsqu'elles sont fonctionnalisées pour des applications à la détection chimique ou biologiques, la distribution des ligands à leur surface n'est pas homogène. Par ailleurs, elles sont parfois difficiles à fonctionnaliser, et cette fonctionnalisation relève d'un procédé différent selon le choix du matériau qui les constitue.

La demande internationale précitée WO2015/055809 évoque la possibilité d'utiliser du graphène pour la réalisation d'une couche antireflet absorbante. Toutefois, pour satisfaire les conditions d'extinction de la réflexion sur un substrat en verre ou silice fondue, il serait nécessaire d'utiliser une couche comprenant au moins 8 à 10 feuillets de graphène. Or, il est très difficile d'obtenir de telles couches avec un niveau d'uniformité acceptable pour l'application considérée, c'est-à-dire sur une étendue supérieure ou égale à 10 micromètres carrés environ.

L'invention vise à surmonter les inconvénients précités des couches antireflets absorbantes connues de l'art antérieur.

Conformément à l'invention, ce but est atteint par l'utilisation d'un revêtement absorbant selon la partie caractérisante de la revendication 1.

La couche sensible, constituée de quelques feuillets d'un matériau bidimensionnel, par exemple de type graphène, permet d'obtenir une surface lisse à l'échelle atomique et, en cas de fonctionnalisation, une distribution homogène des ligands. La sous-couche de contraste est nécessaire pour satisfaire les conditions d'extinction de la réflexion, ce qui ne serait pas possible en utilisant seulement la couche sensible. Par ailleurs, la structure composite du revêtement selon l'invention fournit des degrés de liberté additionnels, rendant plus facile l'obtention d'une extinction de la réflexion pratiquement parfaite.

Il convient de noter que l'utilisation d'une couche de graphène, ou d'un matériau de type graphène, déposée sur une couche métallique a déjà été proposée dans le contexte des capteurs à résonance plasmonique de surface (SPR de l'anglais « Surface Plasmon Resonance »), voir par exemple :

- US 2015/0301039 ; et
- Hoang Hiep Nguyen et al. « Surface Plasmon Resonance: A Versatile Technique for Biosensor Applications » Sensors 2015, 15, 10481-10510.

Cependant ces capteurs exploitent un effet physique tout à fait différent de celui à la base de l'invention et nécessitent des conditions d'utilisation plus contraignantes (éclairage en incidence très oblique et lumière polarisée).

Ainsi, un objet de l'invention est un support amplificateur de contraste pour l'observation d'un échantillon selon selon la revendication 1 annexée.

Un autre objet de l'invention est un procédé de fabrication d'un tel support amplificateur de contraste, selon la revendication 12 annexée. edit procédé comprenant une phase de conception dudit support et une phase de fabrication matérielle du support ainsi conçu.

Encore un autre objet de l'invention est un procédé d'observation d'un échantillon selon la revendication 13 annexée.

Encore un autre objet de l'invention est un procédé de détection ou dosage d'au moins une espèce chimique ou biologique ou des nanoparticules selon la revendication 14 annexée.

D'autres caractéristiques, détails et avantages de l'invention ressortiront à la lecture de la description faite en référence aux dessins annexés donnés à titre d'exemple et qui représentent, respectivement :

- La figure 1, une illustration du principe de fonctionnement d'une couche antireflet absorbante selon l'art antérieur ;
- Les figures 2A, 2B et 2C, des résultats expérimentaux démontrant la faisabilité de l'invention et la réalité de son effet technique ; et
- La figure 3, un support amplificateur de contraste utilisant une couche antireflet absorbante selon un mode de réalisation de l'invention ; et
- Les figures 4A à 4E, différents modes de réalisation de l'invention.

Les figures ne sont pas à l'échelle. En particulier l'épaisseur de certaines couches (et notamment de la couche sensible en matériau de type graphite) est très fortement agrandie dans un souci de clarté.

Dans la suite :

- « Matériau tridimensionnel » désignera un matériau se présentant sous la forme d'un feuillet unique d'épaisseur monoatomique ou monomoléculaire, ou d'un empilement d'un faible nombre (10 au maximum) de tels feuillets, éventuellement de natures différentes ; un matériau bidimensionnel peut être cristallin ou amorphe. Des exemples de matériaux bidimensionnels sont le graphène et les matériaux de type graphène (voir ci-dessous) mais également d'autres matériaux de nature chimique différente tels que WS$_2$, MoS$_2$, WSe$_2$, les polymères bidimensionnels conducteurs, etc. Voir en particulier l'article de Hua Zhang « Ultrathin Two-Dimensional Nanomaterials », ACS Nano, Vol. 9, No. 10, 9451 - 9469 (2015) et, plus spécifiquement pour les polymères bidimensionnels conducteurs l'article de J-J. Adjizian et al. « Dirac Cones in two-dimensional conjugated polymer networks » Nat. Commun.5:5842 18 décembre 2014.
- « Graphène », sans plus de précisions, désignera un feuillet unique (c'est-à-dire une couche d'épaisseur monoatomique) de carbone pur, formant une maille hexagonale ;
- Un « matériau de type graphène » (« Graphene-Related Material, ou GRM, dans la littérature en langue anglaise) désignera soit du graphène pur, soit un feuillet unique d'un matériau dérivé du graphène tel que de l'oxyde de graphène, de l'oxyde de graphène réduit, du graphène dopé par substitution de certains atomes de carbone par d'autres atomes, du graphane, du graphine, etc. ; soit un empilement d'un faible nombre (10 au maximum) de tels feuillets, éventuellement de natures différentes. L'oxyde de graphène est particulièrement intéressant parce qu'on peut le manipuler facilement en solution, parce qu'il est "naturellement" fonctionnalisé avec une haute densité de groupes epoxy, carboxyl et hydroxyl et parce qu'il se prête particulièrement bien à toutes sortes de fonctionnalisations de surface, par exemple par fixation de groupes amines sur les groupes carboxyliques. Voir à ce propos l'article de Nan-Fu Chiu et al. « Graphene Oxide Based Surface Plasmon Resonance Biosensors », chapitre 8 de « Advances in Graphene Science », InTech, 2013.
- Un matériau sera considéré absorbant à une longueur d'onde λ lorsque la partie imaginaire de son indice de réfraction à cette longueur d'onde est supérieure ou égale à 0,0001, de préférence à 0,001, de manière encore préférée à 0,01. Dans le cas contraire, il sera considéré transparent. Avec cette définition, par exemple, tous les TCO (oxydes transparents conducteurs) sont transparents.

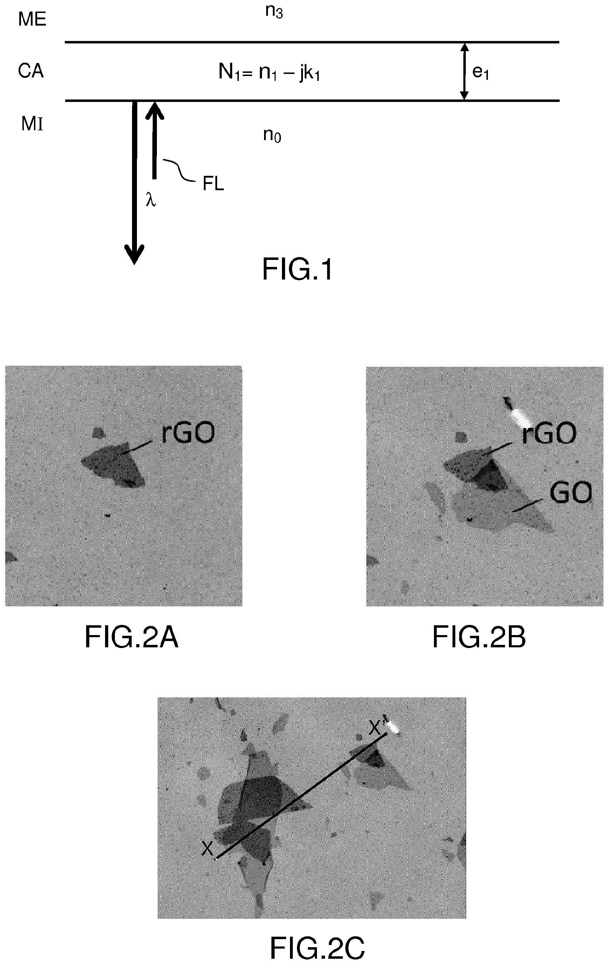

La figure 1 permet de rappeler la théorie des couches antireflet absorbantes, déjà exposée dans la demande internationale précitée WO2015/055809. Elle illustre un faisceau de lumière parallèle FL (pouvant être assimilé localement à une onde plane) et monochromatique à une longueur d'onde (dans le vide) λ, en incidence normale sur une structure constituée par : un milieu semi-infini dit incident MI, dont provient le faisceau de lumière, transparent et caractérisé par un indice de réfraction réel $n_0$ ; une couche absorbante CA d'épaisseur $e_1$, caractérisée par un indice de réfraction complexe $N_1 = n_1 - jk_1$ (« j » étant l'unité imaginaire) ; et un milieu semi-infini dit émergent, ou milieu ambiant, ME, situé du côté de la couche opposé à celui dont provient la lumière, transparent et caractérisé par un indice de réfraction réel $n_3 < n_0$. Le milieu incident peut notamment être un substrat, par exemple en verre, sur lequel est déposée la couche CA. Un échantillon (non représenté) d'indice de réfraction réel $n_2$ - ou d'indice de réfraction complexe $N_2 = n_2 - jk_2$ - peut être déposé sur la couche CA, du côté du milieu émergent. Comme cela a été expliqué plus haut, afin de maximiser le contraste avec lequel l'échantillon est observé, il faut annuler la réflectance de l'ensemble milieu incident MI / couche CA / milieu émergent ME en l'absence d'échantillon.

Le coefficient de réflexion complexe d'une structure du type illustré sur la figure 1 (couche d'épaisseur $e_1$ comprise entre deux milieux semi-infinis) est donné par la formule d'Airy :

$$r_{013} = \frac{r_{01} + r_{13} e^{-2j\beta_1}}{1 + r_{01} r_{13} e^{-2j\beta_1}} \qquad (2)$$

où $r_{ij}$ est le coefficient de Fresnel à l'interface i-j (i,j=0, 1 ou 3, « 0 » correspondant au milieu incident, « 1 » à la couche CA et « 3 » au milieu émergent) et $\beta_1 = 2\pi n_1 e_1 \cos\theta_1 / \lambda$ est le facteur de phase associé à ladite couche, $\theta_1$ étant l'angle de réfraction dans la couche. Dans un premier temps, on considère une couche transparente d'indice réel $n_1$, la généralisation au cas d'une couche absorbante sera traitée plus loin. Toujours dans un premier temps, on considère une incidence qui peut ne pas être normale.

Les coefficients de Fresnel pour les polarisations « p » (TM) et « s » (TE) sont :

$$r_{ij}^{(p)} = \frac{\left(n_j \cos\theta_i - n_i \cos\theta_j\right)}{\left(n_j \cos\theta_i + n_i \cos\theta_j\right)}$$

et

$$r_{ij}^{(s)} = \frac{(n_i \cos\theta_i - n_j \cos\theta_j)}{(n_i \cos\theta_i + n_j \cos\theta_j)}$$

La condition antireflet correspond à $r_{013}=0$ ce qui, dans le cas de milieux transparents (indices réels) donne deux familles de solutions :

- les couches dites « λ/2 », pour lesquelles $e_1 = \dfrac{m\lambda}{(2n_1\cos\theta_1)}$ où m est un entier, qui existent seulement si $n_0=n_3$ ; et

- les couches dites « λ/4 », pour lesquelles $n_1 e_1 = (2p+1)\dfrac{\lambda}{4}$ (p entier).

Dans le cas où le milieu 1 (couche CA) est absorbant, son indice de réfraction $N_1=n_1-jk_1$ est complexe ; l'angle de réfraction - que l'on indique alors par $\Theta_1$ - et le coefficient de phase - $B_1$ - sont également complexes. Dans ce cas, $r_{013}=0$ impose : $r_{01,s}\cdot r_{13,p}= r_{01,p}\cdot r_{13,s}$ ; cette égalité ne peut être vraie que si une des trois conditions suivantes : $\Theta_1=0$ (incidence normale), $N_1{}^2=n_0{}^2$ (pas de couche) ou $n_0{}^2=n_3{}^2$ (milieux incident et émergent identiques) est satisfaite. Par conséquent, dans le cas de milieux extrêmes quelconques, la condition antireflet ne peut être satisfaite qu'en incidence normale. Sachant que $r_{011,p}=-r_{01,s}$ et que $r_{13,p}=-r_{13,s}$, l'équation (2) devient :

$$N_1{}^2 - j\frac{(n_3-n_0)}{tanB_1}N_1 - n_0 n_3 = 0 \qquad (3)$$

L'équation (3) est transcendante et n'admet pas de solution analytique. Toutefois, on peut trouver des solutions correspondant aux cas extrêmes : couche fortement absorbante et couche faiblement absorbante.
Dans le cas fortement absorbant, on peut supposer que $e_1 \ll \lambda$ car la lumière ne se propagerait pas à travers une couche très absorbante et épaisse ; par conséquent, $|B_1| \ll 1$ et on peut alors écrire, au second ordre en $B_1$ : $\quad tanB_1 \cong B_1 = \sqrt{n_3/n_0}\left(N_1/\sqrt{n_0 n_3}\right)\delta_1$, où $\delta_1 = (2\pi n_0/\lambda)e_1$. Il est utile de séparer les parties réelles et imaginaires de l'équation, et d'utiliser les variables « réduites » $v_1 = n_1/\sqrt{n_0 n_3}$ and $\kappa_1 = k_1/\sqrt{n_0 n_3}$. L'équation (3) peut alors s'écrire sous la forme du système suivant :

$$v_1{}^2 = 1 + \kappa_1{}^2 \qquad (4a)$$

$$\delta_1 = \frac{\left(\frac{n_0}{n_3}-1\right)}{2v_1\kappa_1} \qquad (4b)$$

Etant donné que $\delta_1$ doit être réel et positif, on a la condition $n_0>n_3$ (« géométrie inversée »). En prenant $n_0=1,52$ et $n_3=1,34$ - ce qui correspond au cas verre/eau couramment utilisé en biophotonique - on trouve une épaisseur $e_1 = (\lambda/2\pi)(n_0- n_3)/2n_1 k_1$ de l'ordre du nanomètre, ce qui confirme l'hypothèse initiale. Il est intéressant - et inattendu - que l'équation (4a) tend à la condition d'indice classique quand $\kappa_1$ - et donc $k_1$ - tend vers zéro. Une comparaison avec des résultats numériques permet de vérifier que l'équation (4a), bien que dérivée dans l'hypothèse d'une couche fortement absorbante, est approximativement valable pour toute valeur de $k_1$. Par contre, la valeur de $e_1$ obtenue à partir de l'équation (4b) ne tend pas vers $\lambda/4n_1$ ; par conséquent, l'équation (4b) n'a pas une validité générale.
Dans le cas faiblement absorbant on pose $B_1=\pi/2-\varepsilon_1$ (où si est une variable complexe), ce qui implique :

$\varepsilon_1 = \pi/2 - \sqrt{\frac{n_3}{n_0}}(v_1 - j\kappa_1)\delta_1$. On peut alors écrire, au second ordre en $\kappa_1$ :

$$v_1{}^2 = 1 + \frac{\pi}{2}\sqrt{\frac{n_3}{n_0}}\left(\frac{n_0}{n_3}-1\right)\kappa_1 - 3\kappa_1{}^2 + o(\kappa_1{}^3) \qquad (5a)$$

$$\delta_1 \simeq \frac{\pi}{2} \sqrt{\frac{n_0}{n_3}} \frac{1}{v_1} \left\{ 1 - \frac{4}{\pi} \frac{\sqrt{n_0/n_3}}{(n_0/n_3-1)} \kappa_1 + \kappa_1{}^2 + o(\kappa_1{}^3) \right\} \qquad (5b)$$

En pratique, l'équation (5a) - dont le domaine de validité s'avère très réduit - a peu d'intérêt car, comme mentionné plus haut, l'équation (4a) constitue une approximation satisfaisante pour toute valeur de $\kappa_1$. En calculant la solution numérique de l'équation 3 on peut vérifier que la solution obtenue pour $\kappa_1$ élevé ne constitue pas une approximation acceptable pour $\kappa 1$ pour $\kappa_1$ petit. Par contre, il existe une équation semi-empirique qui s'avère satisfaisante dans tous les cas et qui est donnée par l'équation 6b ci-dessous ; l'équation 6a est simplement l'équation 4a qui, comme cela a été montré plus haut, peut être considérée générale et utilisée en remplacement de 5b même pour $\kappa_1$ petit :

$$v_1{}^2 = 1 + \kappa_1{}^2 \qquad (6a)$$

$$\delta_1 \cong \frac{(n_0/n_3-1)}{2v_1\kappa_1} \left[ 1 - e^{-\frac{\kappa_1}{K}} \right] \qquad (6b).$$

où

$$K = \left\{ [\pi/(n_0/n_3 - 1)] \sqrt{n_0/n_3} \right\}^{-1}$$

En pratique, bien qu'étant moins contraignante que la condition 1a qui s'applique aux couches antireflet transparentes la condition 6a reste très contraignante. En pratique, on peut souvent se contenter de satisfaire la condition 6b de manière approchée. Dans le cas où la sous-couche de contraste est réalisée en un matériau relativement peu absorbant ($k_1 < 0{,}15$), la tolérance sur $e_1$ pourra être de l'ordre de $\lambda/100$, voire $\lambda/50$, voire $\lambda/20$, voire $\lambda/10$, voire même $\lambda/5$ ; en termes de $e_1$ cela se traduit par une tolérance de l'ordre de 0,01, 0,02, 0,05, 0,1 voire 0,2. La tolérance sur $e_1$ pourra être beaucoup plus élevée dans le cas où la sous-couche de contraste est réalisée en un matériau qu'on peut considérer très absorbant ($k_1 > 0{,}15$). Dans ce cas, elle pourra atteindre 0,3 voire même 0,6, même si elle sera de préférence de l'ordre de 0,1 ou même de 0,01.

Il est donc particulièrement utile d'introduire une hiérarchie entre les conditions 6a et 6b, qui est justifiée par l'analyse suivante :

L'interférence destructive entre les faisceaux réfléchis par les deux surfaces f' une couche antireflet absorbante résulte de deux contributions :

  i) l'amplitude des coefficients de réflexion de ces deux surfaces ;
  ii) le déphasage entre les faisceaux réfléchis par ces deux surfaces, auquel contribue la différence de déphasage à la réflexion par chacune des deux surfaces et le trajet optique qui les sépare.

L'équation 6a traduit que l'amplitude des coefficients de réflexion est identique : L'équation 6b traduit que les faisceaux émergents sont en opposition de phase.

Si les deux équations sont simultanément satisfaites, l'extinction est totale.

Si seule l'équation 6a est satisfaite, les fluctuations de l'intensité réfléchie par la couche avec son épaisseur sont maximales, ce qui garantit la possibilité d'une extinction parfaite, mais ne garantit pas l'extinction, pas même une extinction satisfaisante.

Si seule l'équation 6b est satisfaite, l'extinction n'est pas forcément parfaite, parce que les amplitudes réfléchies par les deux surfaces peuvent être différentes, mais l'épaisseur de la couche est telle que l'extinction est satisfaisante parce que le chemin optique est réglé pour que ces deux réflexions soient en opposition de phase ou "presque" en opposition de phase. On pourra parler dans ce cas d'une couche anti-résonante. Le mot "presque" traduit le fait que les écarts à la condition 6a affectent les phases des amplitudes réfléchies par les deux surfaces, et qu'ils les affectent faiblement. C'est pourquoi il est plus important d'approcher la condition 6b que d'approcher la condition 6a. En pratique, l'indice $N_1 = n_1 - jk_1$ varie beaucoup avec la longueur d'onde $\lambda$ et le produit $v_1\kappa_1$ parcourt un intervalle [p1, p2]. Il faut donc choisir pour quelle longueur d'onde cible on optimise l'épaisseur de la couche. Les variations du produit $v_1\kappa_1$ avec la longueur d'onde suffisent à rattraper par le choix de la longueur d'onde de travail l'erreur faite sur l'épaisseur en utilisant l'équation 6b avec la longueur d'onde cible quand l'équation 6a n'est pas vérifiée.

Il est donc particulièrement judicieux :

EP 3 391 027 B1

i) de s'approcher de la condition 6a autant que faire se peut (par exemple avec une tolérance inférieure ou égale à 20%, de manière préférée inférieure ou égale 5%, et de manière encore préférée inférieure ou égale à 1%, pour $n_1$ et $k_1$, mais on sera parfois amenés à accepter des tolérances plus élevées) compte tenu des contraintes imposées sur les matériaux;

ii) de choisir une longueur d'onde cible telle que le produit $v_1 \kappa_1$ correspondant se situe vers le milieu de l'intervalle [p1, p2];

iii) de déterminer l'épaisseur réduite $\delta_1$ de la couche au moyen de l'équation 6b appliquée avec cette longueur d'onde cible.

iv) de choisir la longueur d'onde de travail qui procure la meilleure extinction

Dans le cas d'une sous-couche de contraste, et dans le but d'obtenir une sensibilité encore plus grande, il pourra être avantageux de choisir l'épaisseur de la sous-couche en dessous de l'épaisseur idéale donnée par l'équation 6b, par exemple la moitié de cette épaisseur. On pourra parler dans ce cas d'une sous-couche sous-anti-résonante.

Enfin, il est utile de préciser que les conditions antireflet décrites par les équations 6a et 6b, qui sont relatives à l'incidence normale, permettent aussi d'obtenir une réflectivité très faible pour des ouvertures importantes de l'éclairage (par exemple jusqu'à un demi-angle d'ouverture de 60°) et surtout de l'observation, si bien qu'elles permettent une imagerie de grande sensibilité à haute résolution optique.

Les inventeurs se sont rendu compte que les performances d'une couche antireflet absorbante non idéale, c'est-à-dire vérifiant de manière exacte ou approchée la condition 6b (couche « anti-résonante ») mais pas la condition 6a, par exemple en métal, peuvent être améliorées en déposant au-dessus d'elle un petit nombre (entre 1 et 5) de feuillets d'un matériau de type graphène. Cela est illustré par les figures 2A à 2C qui montrent des feuillets d'oxyde de graphène (GO) et oxyde de graphène réduit (rGO) sur une couche antireflet absorbante « optimale » en Cr/Au sur substrat en verre. L'observation est effectuée dans les conditions de la figure 1 ; on peut voir que les régions recouvertes de GO ou rGO apparaissent plus sombres que celles qui en sont dépourvues (contraste négatif) ; cela signifie que la présence d'un matériau de type graphène permet de s'approcher d'avantage d'une condition d'extinction de la réflexion que ce qui est possible en n'utilisant qu'une couche métallique.

L'invention met à profit cette découverte, en proposant un revêtement antireflet absorbant comprenant une sous-couche absorbante « anti-résonante » ou « sous-anti-résonante » réalisée en un matériau autre qu'un matériau bidimensionnel, portant une couche superficielle en matériau bidimensionnel, et notamment de type graphène. Comme cela a été évoqué plus haut, cela permet à la fois d'obtenir une meilleure extinction de la réflexion et de profiter des excellentes propriétés physiques et chimiques des matériaux de type graphène, ou d'autres matériaux bidimensionnels, dont l'utilisation exclusive pour réaliser des couches antireflet absorbantes s'avère délicate.

De manière avantageuse, une sous-couche absorbante selon l'invention présente un comportement antireflet proche de l'optimalité, avec une réflectivité en incidence normale inférieure ou égale à 1%, de manière préférée inférieure ou égale à 0,5% et de manière encore préférée inférieure ou égale à 0,1% voire à 0,05% à la longueur d'onde cible (ou plus généralement une longueur d'onde de l'étendue spectrale de l'éclairage) λ. Par ailleurs, son coefficient de transmission en incidence normale sera de préférence supérieur ou égal à 80% de préférence à 85%, de manière encore préférée à 90% ce qui peut être obtenu, en particulier, grâce à une épaisseur faible, avantageusement inférieure ou égale à 10 nm.

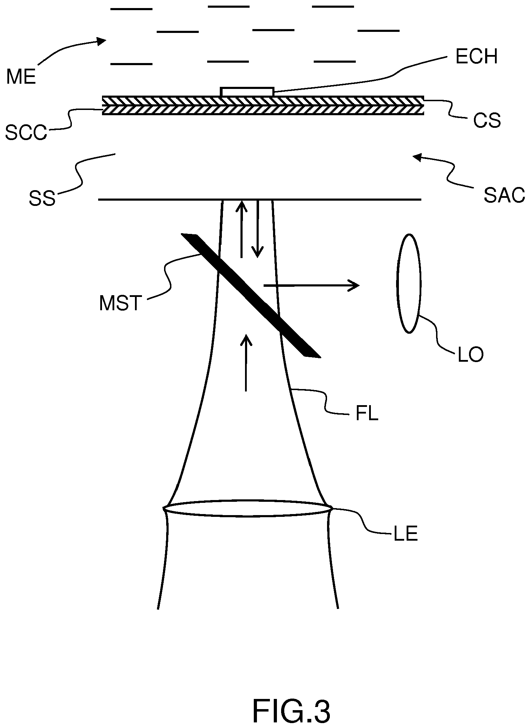

La figure 3 représente un support amplificateur de contraste SAC comprenant un substrat transparent SS - par exemple en verre ou en plastique transparent tel qu'un polycarbonate ou un polystyrène - servant de milieu incident, une sous-couche absorbante antireflet SCC, dite sous-couche de contraste (qui peut en fait présenter une structure composite, en particulier être constituée d'un empilement multicouche), déposée sur le dit substrat, une couche absorbante CS en matériau de type graphène, dite couche sensible, déposée sur la sous-couche de contraste et en contact avec un milieu émergent, ou milieu ambiant, ME, par exemple une solution aqueuse ou l'air. Un échantillon ECH est déposé sur une portion de la couche sensible CS, du côté du milieu émergent. Le substrat est éclairé en incidence normale par un faisceau lumineux FL qui est, dans l'exemple considéré ici, un faisceau laser à profil gaussien, focalisé par une lentille LE au niveau de la couche antireflet. On sait en effet que, dans sa région focale (« beam waist »), un faisceau gaussien présente un front de phase plan, et peut donc être assimilé localement à une onde plane (cas considéré dans les développements théoriques qui précèdent). Un miroir semi-transparent MST dévie une portion de la lumière réfléchie par l'ensemble substrat SS/ sous-couche de contraste SCC/ couche sensible CS/ échantillon ECH/ milieu émergent ME, pour la diriger vers un objectif LO, permettant l'observation dudit échantillon. Dans cette configuration, l'utilisation d'un disque diffusant tournant sur le faisceau laser incident permet d'éliminer les interférences parasites de type chatoiement (« speckle » en anglais). L'observation peut se faire par balayage, notamment à l'aide d'un dispositif de microscopie confocale ou « plein champ ». En variante, il est possible d'utiliser un large faisceau de lumière parallèle pour l'éclairage et d'observer les modulations d'intensité ou de couleur sur la section du faisceau réfléchi ou par imagerie au moyen d'un système de vision télécentrique. Dans la plupart des cas on utilisera un cône d'éclairage ayant un demi-angle d'ouverture pouvant atteindre 60°, mais souvent de l'ordre de 20° - 30° ou moins, centré autour de la direction

7

d'incidence normale ou, en tout cas, incluant cette direction.

Il convient de noter que la cohérence spatiale de la lumière incidente et son état de polarisation n'ont pas d'importance ; le plus souvent l'éclairage sera donc non polarisé. En revanche, si on veut observer et/ou mesurer un contraste d'intensité, il convient d'utiliser un éclairage à bande étroite, ou constitué de plusieurs bandes spectrales étroites disjointes ; un éclairage polychromatique conduisant à un contraste qui n'est pas tant d'intensité que de couleur (échantillon observé avec une couleur différente de celle du fond et couleurs différentes selon l'épaisseur ou l'indice de l'échantillon).

Dans le montage de la figure 3, les lentilles LO et LE sont interchangeables. Par ailleurs, la réflexion parasite sur la face avant du substrat peut être utilement atténuée par des techniques telles que : immersion dans une huile, l'existence d'un biseau entre la face avant et la face arrière, un filtrage spatial, un traitement antireflet classique.

Le support amplificateur de contraste selon l'invention peut aussi être utilisé en microscopie de fluorescence ou de diffusion Raman. Dans ce cas, ses propriétés antireflet servent à atténuer très fortement la réflexion à la longueur d'onde d'éclairage pour faciliter la détection du rayonnement de fluorescence ou diffusion Raman, moins intense.

Pour concevoir un support amplificateur de contraste du type illustré sur la figure 3 on peut procéder de la façon suivante :

- Premièrement, on choisit un premier matériau destiné à constituer le substrat et un matériau destiné à constituer le « milieu ambiant » ou « émergent ». Souvent, le choix du milieu ambiant est déterminé par l'application considérée (généralement une solution aqueuse pour les applications biologiques) ; le choix du matériau constituant le substrat est dicté par des considérations technologiques et par la contrainte $n_3 < n_0$ à la longueur d'onde $\lambda$ utilisée pour l'éclairage et/ou l'observation. Souvent, on choisira un substrat en verre ou en plastique transparent, et un milieu ambiant constitué par l'air (rapport $n_3/n_0$ compris entre 1,45 et 1,7) ou l'eau (rapport $n_3/n_0$ compris entre 1,1 et 1,3).
- Deuxièmement on détermine la longueur d'onde d'éclairage (ou la plus petite longueur d'onde d'éclairage, si ce dernier est polychromatique) $\lambda$, en fonction de l'application considérée ou de différentes contraintes technologiques.
- Troisièmement, l'équation 6a est utilisée pour déterminer la relation liant la partie réelle et la partie imaginaire de l'indice de réfraction du matériau constituant la sous-couche de contraste. Un matériau satisfaisant de manière approchée cette relation est alors choisi - ou conçu. Par exemple, on peut choisir un matériau de départ transparent en fonction de considérations technologiques diverses - par exemple un polymère ; prendre la partie réelle de son indice de réfraction comme une donnée imposée ; et modifier la partie imaginaire dudit indice de réfraction par l'ajout d'impuretés (colorants, nanoparticules...) pour que l'équation 6a soit approchée autant que possible.
- Ensuite l'épaisseur de ladite sous-couche est déterminée en appliquant l'équation 6b (ou l'une des équations 4b ou 5b, qui en constituent des cas particuliers).
- Puis on choisit un matériau bidimensionnel, par exemple de type graphène, destiné à former la couche sensible. Le choix peut être dicté par différentes considérations, optiques (maximisation de l'extinction de la réflexion, on pourra pour cela s'aider de simulations numériques) et/ou physico-chimiques (obtenir une surface lisse au niveau atomique et/ou une interaction particulière avec l'échantillon). La couche sensible peut notamment être fonctionnalisée, c'est-à-dire porter des molécules (ligands) susceptibles de lier certaines espèces chimiques ou biologiques.

On procède ensuite à la fabrication du support, par des techniques conventionnelles, telles que le revêtement à la tournette, par immersion, au rouleau, par sédimentation, ou par évaporation ; le dépôt chimique ou physique en phase vapeur, l'implantation ionique, le dépôt électrolytique, le transfert de Langmuir-Blodgett, méthode de la bulle (J. Azevedo et al. « Versatile Wafer-Scale Technique for the Formation of Ultrasmooth and Thickness-Controlled Graphene Oxide Films Based on Very Large Flakes », ACS Appl Mater Interfaces. 2015 Sep 30;7(38):21270-7), etc.

La sous-couche de contraste peut être métallique (et notamment en or), semi-conductrice, conductrice non-métallique, en polymère contenant des pigments ou colorants, en matériau inorganique (minéral) contenant des centres colorés, etc. Parmi les matériaux semi-conducteurs convenant à la réalisation de couches antireflet absorbantes on peut mentionner : le germanium (pour des applications dans l'ultraviolet (UV) proche, par exemple à 354 nm), le $TiO_2$ (également dans l'UV proche), le siliciure de molybdène, de nickel ou de titane (dans le visible), le siliciure de tungstène (dans le proche infrarouge ou dans le proche UV), le siliciure de zirconium (dans le visible ou le proche UV) le tantale ou le vanadium (dans le visible), etc. Elle peut également contenir des nanoparticules métalliques. Elle peut être magnétique, ce qui présente un intérêt pour l'étude d'échantillons qui sont à leur tour magnétiques. L'utilisation de couches conductrices - métalliques ou pas - permet d'appliquer une différence de potentiel contrôlée à l'échantillon et/ou de réaliser une « imagerie électrochimique » permettant d'étudier des phénomènes d'électrodépôt, corrosion, catalyse, etc, et/ou d'activer/désactiver les ligands de la couche sensible fonctionnalisée. Une variante particulièrement intéressante consiste à réaliser un support monolithique, dans lequel la sous-couche est une couche d'impuretés implantées - par exemple par implantation ionique à basse énergie - à la surface du substrat. Bien que qualifiée de « absorbante », la sous-couche de contraste ne doit pas nécessairement être absorbante au sens propre : en variante, il peut s'agir d'une couche diffusante, la diffusion « imitant » l'absorption et pouvant également être modélisée par un indice de réfraction complexe. En outre, la sous-couche de contraste peut être constituée d'un empilement de couches élémentaires. Enfin, il peut-être avantageux que la sous-couche de contraste soit elle-même fonctionnalisée, par exemple par une couche d'acide mercaptotoctanoïque ($C_8H_{16}O_2S$) dans le cas où c'est une couche d'or (voir à ce propos l'article précité de Hua Zhang).

De nombreuses techniques permettent la fabrication de la couche sensible en matériau de type graphène, telles que le dépôt chimique en phase vapeur (CVD), l'épitaxie par faisceaux moléculaires (MBE) et des techniques en phase liquide. Voir par exemple

- F. Bonaccorso et al. « Production and processing of graphene and 2d crystals » Materials Today, Vol. 15, n° 12 (décembre 2012), pages 564 - 589.

La couche sensible peut être constituée d'un empilement de feuillets d'un même type ou différents.

Un substrat amplificateur de contraste tel que décrit ci-dessus, présentant une couche sensible fonctionnalisée, permet la réalisation de biopuces pour la détection et/ou le dosage d'espèces chimiques ou biologiques ou de nanoparticules, ou encore de nanoparticules véhiculant elles-mêmes des espèces biologiques ou chimiques à détecter capturées par les nanoparticules dans une étape précédente. Dans cette application, l'utilisation d'un éclairage infrarouge ou ultraviolet est particulièrement avantageuse. En effet, la plupart des espèces capturées ont des bandes d'absorption en infrarouge ou en ultraviolet qui permettent de les détecter spécifiquement, autrement dit de les reconnaître. Cette détection spécifique coopère avec la capture spécifique assurée par les ligands, et la renforce en superposant une spécificité à l'autre.

La détection/dosage d'espèces chimiques ou biologiques ou nanoparticules peut se faire directement par capture des espèces à détecter/doser, ou indirectement par remplacement ou disparition d'une espèce préalablement capturée par les ligands.

Afin de faciliter les applications à la détection d'espèces biologiques ou chimiques, incluant les nanoparticules, le support peut utilement constituer le fond d'une boîte de Pétri ou encore d'une cellule fluidique comprenant un ou plusieurs canaux d'un diamètre minimal de 1 micron, permettant une manipulation économique et parfaitement contrôlée des liquides ou des gaz analysés.

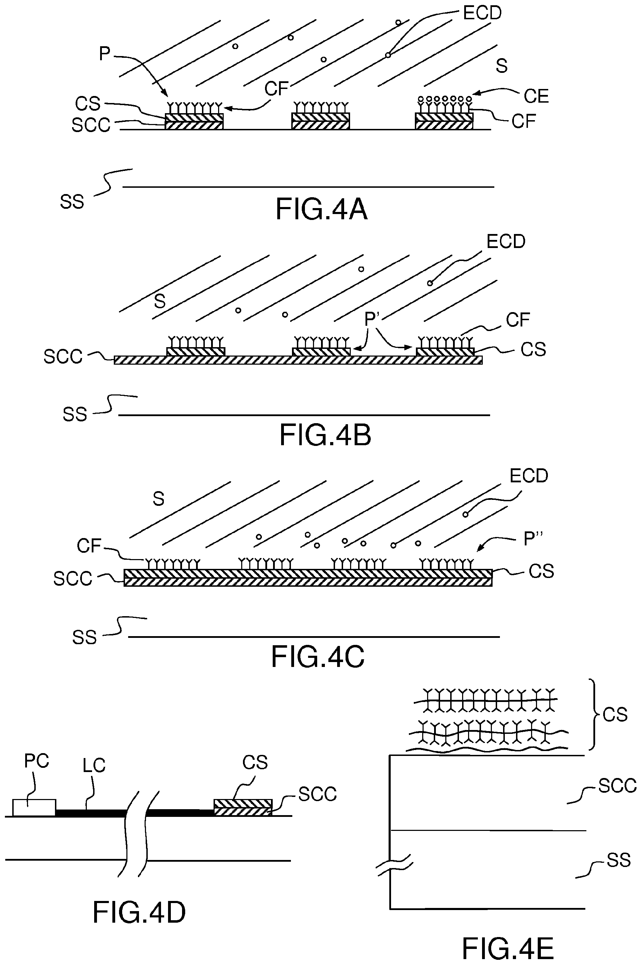

La figure 4A illustre un mode de réalisation dans lequel tant la sous-couche de contraste que la couche sensible sont discontinues et forment des plots P. Une couche fonctionnalisée CF est déposée sur la couche sensible de chaque plot ; typiquement des plots différents recevront des fonctionnalisation différentes permettant de fixer sélectivement des espèces chimiques ou biologiques différentes. Les couches fonctionnalisées sont mises en contact avec une solution S par exemple aqueuse, ou bien avec un gaz, contenant la ou les espèces chimiques ou biologiques à détecter ECD. Ces dernières sont fixées par les couches fonctionnalisées des plots respectifs et forment des couches minces supplémentaires CE, constituant l'échantillon à observer (le cas d'un seul plot est représenté). En observant la biopuce au microscope, dans les conditions décrites ci-dessus, on peut identifier facilement les espèces effectivement présentes dans la solution.

La figure 4B illustre une variante dans laquelle la sous-couche de contraste est continue, et les plots P' sont formés par une couche sensible CS discontinue.

La figure 4C illustre une autre variante dans laquelle aussi bien la sous-couche de contraste que la couche sensible sont continues, et les plots P" sont définis uniquement par des couches de fonctionnalisation CF localisées. Dans ce cas il peut être utile de prévoir, en dehors des plots, une couche de passivation empêchant la fixation de toute espèce chimique ou biologique contenue dans ladite solution (« passivation chimique »). On peut utiliser par exemple un polyéthylène glycol, un polymère fluoré, ou un alkyl fluoré.

La figure 4D illustre schématiquement le cas où la sous-couche de contraste est métallique (ou plus généralement conductrice) et connectée par l'intermédiaire d'une ligne conductrice LC à un plot de contact PC permettant l'application d'une tension électrique, par exemple afin d'activer/désactiver de manière sélective des ligands d'une couche de fonctionnalisation ou de favoriser/défavoriser la capture d'une cible chargée par une action électrostatique ou électrocinétique (telle qu'une électrophorèse). L'électro-activation de ligands est décrite, par exemple, dans l'article de Lucian-Gabriel Zamfir et al. « Synthesis and electroactivated addressing of ferrocenyl and azido-modified stem-loop oligonucleotides on an integrated electrochemical device » Electrochimica Acta 164 (2015) 62-70.

La figure 4E illustre schématiquement une couche sensible CS constituée de trois feuillets d'un matériau de type graphène, dont deux présentent une fonctionnalisation sur leurs deux faces. Pour ce faire on peut fonctionnaliser de l'oxyde de graphène en solution, plus le déposer par la méthode de la bulle appliquée à plusieurs reprises. Dans ces conditions, il se produit un phénomène d'intercalation qui démultiplie l'efficacité de la capture des espèces à détecter par les ligands. La sensibilité de la détection en est grandement améliorée.

**Revendications**

1. Support amplificateur de contraste (SAC) pour l'observation d'un échantillon (ECH, ECD), comprenant un substrat transparent (SS) portant au moins un revêtement absorbant adapté pour se comporter en tant que revêtement antireflet lorsqu'il est éclairé en incidence normale à une longueur d'onde d'éclairage $\lambda$ à travers ledit substrat et lorsque la face dudit revêtement opposée audit substrat est en contact avec un milieu dit ambiant (ME) transparent dont l'indice de réfraction $n_3$ est inférieur à celui de l'indice de réfraction $n_0$ dudit substrat, **caractérisé en ce que** ledit revêtement absorbant comprend :

   - une sous-couche absorbante à ladite longueur d'éclairage $\lambda$ et présentant, lorsqu'elle est éclairée en incidence normale à cette même longueur d'onde, une extinction de la réflexion par interférence destructive, dite sous-couche de contraste (SCC), déposée à la surface dudit substrat transparent ; et
   - une couche absorbante dite sensible (CS), distincte de ladite sous-couche de contraste et comprenant entre 1 et 5 feuillets d'au moins un matériau bidimensionnel.

2. Support amplificateur de contraste selon la revendication 1 dans lequel ledit ou au moins un dit matériau bidimensionnel est un matériau de type graphène.

3. Support amplificateur de contraste selon l'une des revendications précédentes dans lequel ledit revêtement absorbant est discontinu, formant une pluralité de plots (P) à la surface dudit substrat.

4. Support amplificateur de contraste selon l'une des revendications 1 ou 2 dans lequel ladite sous-couche de contraste est continue et ladite couche sensible est discontinue, formant une pluralité de plots (P') à la surface dudit substrat.

5. Support amplificateur de contraste selon l'une des revendications précédentes dans lequel ladite sous-couche est conductrice et est reliée par une ligne conductrice (LC) à un plot de contact (PC) permettant l'application d'un potentiel électrique.

6. Support amplificateur de contraste selon l'une des revendications précédentes dans lequel, à ladite longueur d'onde d'éclairage $\lambda$, l'indice de réfraction $n_0$ du substrat, les parties réelles et imaginaires de l'indice de réfraction complexe de la sous-couche de contraste $N_1 = n_1 - jk_1$ et l'épaisseur $e_1$ de la sous-couche de contraste satisfont aux conditions suivantes :

   •

$$\delta_1 = \frac{(n_0/n_3 - 1)}{2\nu_1 \kappa_1} \left[ 1 - e^{-\frac{\kappa_1}{K}} \right] \quad ;$$

   et
   • $k_1 \geq 0{,}001$, et de préférence $k_1 \geq 0{,}01$, et de manière encore préférée $k_1 \geq 0{,}1$
   • $k_1 < 0{,}15$;
   où :

   -

$$\delta_1 = \frac{2\pi n_0}{\lambda} e_1;$$

   -

$$\nu_1 = \frac{n_1}{\sqrt{n_0 n_3}} \quad ;$$

   -

$$\kappa_1 = \frac{k_1}{\sqrt{n_0 n_3}}\,;$$

et

-

$$K = \left\{[\pi/(n_0/n_3 - 1)]\sqrt{n_0/n_3}\right\}^{-1}$$

avec une tolérance inférieure ou égale à 0,2, de préférence inférieure ou égale à 0,1, de préférence inférieure ou égale à 0,02, et de manière encore préférée inférieure ou égale à 0,01 pour $\delta_1$.

7. Support amplificateur de contraste selon l'une des revendications 1 à 5 dans lequel l'indice de réfraction du substrat $n_0$, l'indice de réfraction complexe de la sous-couche de contraste $N_1 = n_1 - jk_1$ et l'épaisseur $e_1$ de la sous-couche de contraste satisfont, en outre, avec ladite tolérance et pour ladite longueur d'onde d'éclairage $\lambda$, aux conditions suivantes :

•

$$\delta_1 = \frac{n_0/n_3 - 1}{2\nu_1 \kappa_1};$$

et
•

$$k_1 \geq 0,15$$

où :

-

$$\delta_1 = \frac{2\pi n_0}{\lambda} e_1;$$

-

$$\nu_1 = \frac{n_1}{\sqrt{n_0 n_3}}\,;$$

-

$$\kappa_1 = \frac{k_1}{\sqrt{n_0 n_3}}\,;$$

et
-

$$K = \left\{[\pi/(n_0/n_3 - 1)]\sqrt{n_0/n_3}\right\}^{-1}$$

avec une tolérance inférieure ou égale à 0,6, de préférence inférieure ou égale à 0,3, de préférence inférieure ou égale à 0,1, et de manière encore préférée inférieure ou égale à 0,01 pour $\delta_1$.

8. Support amplificateur de contraste selon l'une des revendications 6 ou 7 dans lequel, à ladite longueur d'onde d'éclairage $\lambda$, l'indice de réfraction $n_0$ du substrat, les parties réelles et imaginaires de l'indice de réfraction complexe de la sous-couche de contraste $N_1 = n_1 - jk_1$ et l'épaisseur $e_1$ de la sous-couche de contraste satisfont également à la condition suivante :

$$\nu_1{}^2 = 1 + \kappa_1{}^2$$

avec une tolérance inférieure ou égale à 20%, de manière préférée inférieure ou égale 5%, et de manière encore préférée inférieure ou égale à 1%, pour $n_1$ et $k_1$.

9. Support amplificateur de contraste selon l'une des revendications précédentes dans lequel ledit revêtement absorbant antireflet présente en incidence normale une transmittance supérieure ou égale à 80%, de préférence supérieure ou égale à 85% et de manière encore préférée supérieure ou égale à 90% à ladite longueur d'onde $\lambda$.

10. Support amplificateur de contraste selon l'une des revendications précédentes dans lequel ledit revêtement absorbant antireflet présente en incidence normale, à ladite longueur d'onde $\lambda$, une réflectivité inférieure ou égale à 1%, de manière préférée inférieure ou égale à 0,5% et de manière encore préférée inférieure ou égale à 0,1% et de manière encore préférée inférieure ou égale à 0,05%.

11. Support amplificateur de contraste selon l'une des revendications précédentes dans lequel ladite couche sensible est fonctionnalisée par l'adjonction ou la présence naturelle de ligands susceptibles de fixer au moins une espèce chimique ou biologique.

12. Procédé de fabrication d'un support amplificateur de contraste selon l'une des revendications précédentes, ledit procédé comprenant une phase de conception dudit support et une phase de fabrication matérielle du support ainsi conçu ; **caractérisé en ce que** ladite étape de conception comprend :

   • le choix :

   - d'un matériau transparent constituant le substrat dudit support ;
   - d'un matériau absorbant constituant ladite sous-couche absorbante ;
   - d'un milieu ambiant destiné à être mis en contact avec ledit revêtement du côté opposé audit substrat ; et
   - d'une longueur d'onde cible $\lambda$ ;

   • la détermination de l'épaisseur $e_1$ de ladite sous-couche absorbante de manière à satisfaire la condition suivante :

$$\delta_1 = \frac{(n_0/n_3 - 1)}{2\nu_1\kappa_1}\left[1 - e^{-\frac{\kappa_1}{K}}\right]$$

   avec une tolérance inférieure ou égale 0,1, de préférence inférieure ou égale à 0,05, de préférence inférieure ou égale à 0,01, et de manière encore préférée inférieure ou égale à 0,005 pour $\delta_1$; et
   • le choix d'un matériau bidimensionnel constituant la couche sensible dudit support, adapté pour être visualisé avec un contraste négatif lorsqu'il est déposé sur ladite sous-couche de contraste, mis en contact avec ledit milieu ambiant, éclairé à travers ledit substrat à ladite longueur d'onde cible $\lambda$ et observé également à travers ledit substrat.
   où :

   - $n_0$ est l'indice de réfraction réel du substrat à la longueur d'onde cible $\lambda$ ;
   - $N_1 = n_1 - jk_1$ avec $N_1 = n_1 - jk_1$ est l'indice de réfraction complexe de la sous-couche de contraste ;
   - $n_3 < n_0$ est l'indice de réfraction réel du milieu ambiant à la longueur d'onde cible $\lambda$ ;
   -

$$\delta_1 = \frac{2\pi n_0}{\lambda} e_1;$$

$$v_1 = \frac{n_1}{\sqrt{n_0 n_3}} \; ;$$

-

$$\kappa_1 = \frac{k_1}{\sqrt{n_0 n_3}} \; ;$$

e

-

$$K = \left\{ \left[ \pi / \left( \frac{n_0}{n_3} - 1 \right) \right] \sqrt{n_0/n_3} \right\}^{-1} .$$

**13.** Procédé d'observation d'un échantillon comportant les étapes suivantes :

A. Disposer ledit échantillon (ECH) sur la couche sensible d'un support amplificateur de contraste selon l'une des revendications 1 à 11 et le mettre en contact avec un milieu ambiant (ME) transparent dont l'indice de réfraction est inférieur à celui du substrat dudit support amplificateur de contraste ;
B. éclairer ledit échantillon à travers ledit milieu ambiant, avec un cône d'éclairage incluant l'incidence normale, avec un rayonnement lumineux incluant au moins une longueur d'onde $\lambda$ telle que le revêtement absorbant dudit support se comporte comme un revêtement antireflet ; et
C. observer l'échantillon ainsi éclairé, également à travers-ledit milieu ambiant.

**14.** Procédé de détection ou dosage d'au moins une espèce chimique ou biologique ou des nanoparticules comportant les étapes suivantes :

I. Procurer un support amplificateur de contraste selon l'une des revendications 1 à 11 comprenant une couche sensible fonctionnalisée (CF), susceptible de fixer au moins une espèce chimique ou biologique ou des nanoparticules ;
II. Placer ladite couche ou surface fonctionnalisée en contact avec au moins une solution (S) contenant une espèce chimique ou biologique (ECD) susceptible de se fixer sur ladite couche ou surface fonctionnalisée, ladite solution étant sensiblement transparente et présentant un indice de réfraction inférieur à celui du substrat dudit support amplificateur de contraste ;
III. Éclairer ledit échantillon avec un cône d'éclairage incluant l'incidence normale à travers ledit milieu ambiant à une longueur d'onde d'éclairage $\lambda$ telle que le revêtement absorbant dudit support se comporte comme un revêtement antireflet ; et
IV. Observer ledit support amplificateur de contraste ainsi éclairé, également à travers ledit substrat.

**15.** Procédé selon la revendication 14 dans lequel ladite étape IV. est effectuée par détection d'un rayonnement de fluorescence ou de diffusion Raman.

**Patentansprüche**

**1.** Kontrastverstärkungsträger (SAC) zum Beobachten einer Probe (ECH, ECD), umfassend ein transparentes Substrat (SS), das mindestens eine absorbierende Beschichtung trägt, die so ausgelegt ist, dass sie als Antireflexbeschichtung wirkt, wenn sie unter normalem Einfall bei einer Beleuchtungswellenlänge $\lambda$ durch das Substrat hindurch beleuchtet wird und wenn die dem Substrat gegenüberliegende Seite der Beschichtung mit einem sogenannten transparenten Umgebungsmedium (ME) in Kontakt ist, dessen Brechungsindex $n_3$ kleiner als der Brechungsindex $n_0$ des Substrats ist, **dadurch gekennzeichnet, dass** die absorbierende Beschichtung Folgendes umfasst:

- eine absorbierende Unterschicht bei der Beleuchtungslänge $\lambda$, die, wenn sie bei senkrechtem Einfall bei der gleichen Wellenlänge beleuchtet wird, eine Extinktion der Reflexion durch destruktive Interferenz aufweist, die als Kontrastunterschicht (SCC) bezeichnet wird und auf die Oberfläche des transparenten Substrats abgesetzt ist; und

- eine sogenannte empfindliche absorbierende Schicht (CS), die sich von der Kontrastunterschicht unterscheidet und zwischen 1 und 5 Blätter aus mindestens einem zweidimensionalen Material umfasst.

2. Kontrastverstärkungsträger nach Anspruch 1, wobei das oder mindestens ein zweidimensionale(s) Material ein graphenartiges Material ist.

3. Kontrastverstärkungsträger nach einem der vorherigen Ansprüche, wobei die absorbierende Beschichtung diskontinuierlich ist und mehrere Pads (P) auf der Oberfläche des Substrats bildet.

4. Kontrastverstärkungsträger nach Anspruch 1 oder 2, wobei die Kontrastunterschicht kontinuierlich und die empfindliche Schicht diskontinuierlich ist und mehrere Pads (P') auf der Oberfläche des Substrats bildet.

5. Kontrastverstärkungsträger nach einem der vorherigen Ansprüche, wobei die Unterschicht leitfähig ist und durch eine leitfähige Leitung (LC) mit einer Kontaktstelle (PC) verbunden ist, die das Anlegen eines elektrischen Potentials ermöglicht.

6. Kontrastverstärkungsträger nach einem der vorherigen Ansprüche, wobei bei der Beleuchtungswellenlänge $\lambda$ der Brechungsindex $n_0$ des Substrats, die Real- und Imaginärteile des komplexen Brechungsindex der Kontrastunterschicht $N_1 = n_1 - jk_1$ und die Dicke $e_1$ der Kontrastunterschicht die folgenden Bedingungen erfüllen:

$$\bullet \quad \delta_1 = \frac{(n_0/n_3 - 1)}{2v_1\kappa_1}\left[1 - e^{-\frac{\kappa_1}{K}}\right] \quad ;$$

und

$k_1 \geq 0{,}001$, und vorzugsweise $k_1 \geq 0{,}01$ und noch bevorzugter $k_1 \geq 0$, list $k_1 < 0{,}15$ wobei:

-

$$\delta_1 = \frac{2\pi n_0}{\lambda}e_1;$$

-

$$v_1 = \frac{n_1}{\sqrt{n_0 n_3}} \quad ;$$

-

$$\kappa_1 = \frac{k_1}{\sqrt{n_0 n_3}} \quad ;$$

und

-

$$K = \left\{[\pi/(n_0/n_3 - 1)]\sqrt{n_0/n_3}\right\}^{-1}$$

mit einer Toleranz von kleiner oder gleich 0,2, vorzugsweise kleiner oder gleich oder kleiner als 0,1, vorzugsweise gleich oder kleiner als 0,02 und noch bevorzugter gleich oder kleiner gleich 0,01 für $\delta_1$.

7. Kontrastverstärkungsträger nach einem der Ansprüche 1 bis 5, wobei der Brechungsindex des Substrats $n_0$, der komplexe Brechungsindex der Kontrastunterschicht $N_1=n_1-jk_1$ und die Dicke $e_1$ der Kontrastunterschicht ferner mit der Toleranz und für die Beleuchtungswellenlänge $\lambda$ die folgenden Bedingungen erfüllen:

- 

$$\delta_1 = \frac{n_0/n_3 - 1}{2v_1\kappa_1};$$

und
- 

$$k_1 \geq 0,15$$

wobei:

-

$$\delta_1 = \frac{2\pi n_0}{\lambda} e_1;$$

-

$$v_1 = \frac{n_1}{\sqrt{n_0 n_3}};$$

-

$$\kappa_1 = \frac{k_1}{\sqrt{n_0 n_3}};$$

und

-

$$K = \left\{ [\pi/(n_0/n_3 - 1)]\sqrt{n_0/n_3} \right\}^{-1}$$

mit einer Toleranz von kleiner oder gleich 0,6, vorzugsweise kleiner oder gleich 0,3, vorzugsweise kleiner oder gleich 0,1 und noch bevorzugter kleiner oder gleich 0,01 für $\delta_1$.

8. Kontrastverstärkungsträger nach Anspruch 6 oder 7, wobei bei der Beleuchtungswellenlänge $\lambda$ der Brechungsindex $n_0$ des Substrats, der Real- und Imaginärteil des komplexen Brechungsindex der Kontrastunterschicht $N_1=n_1-jk_1$ und die Dicke $e_1$ der Kontrastunterschicht auch die folgende Bedingung erfüllen:

$$v_1{}^2 = 1 + \kappa_1{}^2$$

mit einer Toleranz von kleiner oder gleich 20%, vorzugsweise kleiner oder gleich 5% und noch bevorzugter kleiner oder gleich 1%, für $n_1$ und $k_1$.

9. Kontrastverstärkungsträger nach einem der vorherigen Ansprüche, wobei die absorbierende Antireflexionsbeschichtung bei normalem Einfall einen Transmissionsgrad größer oder gleich 80%, vorzugsweise größer oder gleich 85% und noch bevorzugter größer oder gleich 90% bei der Wellenlänge λ aufweist.

10. Kontrastverstärkungsträger nach einem der vorherigen Ansprüche, wobei die absorbierende Antireflexionsbeschichtung bei normalem Einfall bei der Wellenlänge λ ein Reflexionsvermögen von kleiner oder gleich 1%, vorzugsweise kleiner oder gleich 0,5% und noch bevorzugter kleiner oder gleich 0,1% und noch bevorzugter kleiner oder gleich 0,05% aufweist.

11. Kontrastverstärkungsträger nach einem der vorherigen Ansprüche, bei dem die empfindliche Schicht durch Zugabe oder natürliches Vorhandensein von Liganden funktionalisiert ist, die mindestens eine chemische oder biologische Spezies fixieren können.

12. Verfahren zur Herstellung eines Kontrastverstärkungsträgers nach einem der vorherigen Ansprüche, wobei das Verfahren einen Schritt des Entwerfens des Trägers und einen Schritt der materiellen Herstellung des so entworfenen Trägers beinhaltet; **dadurch gekennzeichnet, dass** der Schritt des Entwerfens Folgendes beinhaltet:

   • Wählen:

   - eines transparenten Materials, das das Substrat des Trägers bildet;
   - eines absorbierenden Materials, das die absorbierende Unterschicht bildet;
   - eines Umgebungsmediums, das dazu bestimmt ist, mit der Beschichtung auf der dem Substrat gegenüberliegenden Seite in Kontakt gebracht zu werden; und
   - einer Zielwellenlänge λ;

   • Bestimmen der Dicke $e_1$ der absorbierenden Unterschicht, so dass die folgende Bedingung erfüllt ist:

$$\delta_1 = \frac{(n_0/n_3 - 1)}{2\nu_1\kappa_1}\left[1 - e^{-\frac{\kappa_1}{K}}\right]$$

   mit einer Toleranz von kleiner oder gleich 0,1, vorzugsweise gleich kleiner oder 0,05, vorzugsweise kleiner oder gleich 0,01 und noch bevorzugter kleiner oder gleich 0,005 für $\delta_1$; und
   • Wählen eines zweidimensionalen Materials, das die empfindliche Schicht des Trägers bildet, so ausgelegt, dass es mit negativem Kontrast betrachtet werden kann, wenn es auf die Kontrastunterschicht abgesetzt, mit dem Umgebungsmedium in Kontakt gebracht, durch das Substrat mit der Zielwellenlänge λ beleuchtet und auch durch das Substrat beobachtet wird,
   wobei:

   - $n_0$ ist der reale Brechungsindex des Substrats bei der Zielwellenlänge λ ist;
   - $N_1 = n_1 - jk_1$, wobei $N_1 = n_1 - jk_1$ der komplexe Brechungsindex der Kontrastunterschicht ist;
   - $n_3 < n_0$ der reale Brechungsindex des Umgebungsmediums bei der Zielwellenlänge λ ist;
   -

$$\delta_1 = \frac{2\pi n_0}{\lambda}e_1;$$

   -

$$\nu_1 = \frac{n_1}{\sqrt{n_0 n_3}};$$

   -

$$\kappa_1 = \frac{k_1}{\sqrt{n_0 n_3}};$$

und

-

$$K = \left\{ \left[ \pi / \left( \frac{n_0}{n_3} - 1 \right) \right] \sqrt{n_0/n_3} \right\}^{-1}.$$

13. Verfahren zum Beobachten einer Probe, das die folgenden Schritte beinhaltet:

A. Anordnen der Probe (ECH) auf der empfindlichen Schicht eines Kontrastverstärkungsträgers nach einem der Ansprüche 1 bis 11 und Inkontaktbringen derselben mit einem transparenten Umgebungsmedium (ME), dessen Brechungsindex niedriger ist als der des Substrats des Kontrastverstärkungsträgers;
B. Beleuchten der Probe durch das Umgebungsmedium mit einem Beleuchtungskegel, der den normalen Einfall einschließt, mit einer Lichtstrahlung, die mindestens eine Wellenlänge $\lambda$ umfasst, so dass sich die absorbierende Beschichtung des Trägers wie eine Antireflexbeschichtung verhält; und
C. Beobachten der so beleuchteten Probe auch durch das Umgebungsmedium.

14. Verfahren zum Nachweisen oder Dosieren mindestens einer chemischen oder biologischen Spezies oder von Nanopartikeln, das die folgenden Schritte beinhaltet:

I. Bereitstellen eines Kontrastverstärkungsstägers nach einem der Ansprüche 1 bis 11, der eine funktionalisierte empfindliche Schicht (CF) umfasst, die mindestens eine chemische oder biologische Spezies oder Nanopartikel fixieren kann;
II. Inkontaktbringen der Schicht oder funktionalisierten Oberfläche mit mindestens einer Lösung (S), die eine chemische oder biologische Spezies (ECD) enthält, die sich an der Schicht oder der funktionalisierten Oberfläche fixieren kann, wobei die Lösung im Wesentlichen transparent ist und einen Brechungsindex aufweist, der niedriger ist als der des Substrats des Kontrastverstärkerträgers;
III. Beleuchten der Probe mit einem Beleuchtungskegel, der den normalen Einfall einschließt, durch das Umgebungsmedium bei einer Beleuchtungswellenlänge $\lambda$, so dass sich die absorbierende Beschichtung des Mediums wie eine Antireflexbeschichtung verhält; und
IV. Beobachten des so beleuchteten Kontrastverstärkungsmittels auch durch das Substrat.

15. Verfahren nach Anspruch 14, wobei Schritt IV. durch Nachweisen von Raman-Fluoreszenz- oder -Streustrahlung durchgeführt wird.

**Claims**

1. A contrast-amplifying carrier (SAC) for observing a sample (ECH, ECD), comprising a transparent substrate (SS) bearing at least one absorbent coating suitable for behaving as an antireflection coating when it is illuminated at normal incidence at an illumination wavelength $\lambda$ through said substrate and when the face of said coating opposite said substrate is in contact with a medium referred to as a transparent ambient medium (ME), the refractive index $n_3$ of which is lower than that of the refractive index $n_0$ of said substrate, **characterized in that** said absorbent coating comprises:

an absorbent sublayer at said illumination wavelength $\lambda$ and which exhibits, when it is illuminated at normal incidence at this same wavelength, a reflection extinction by destructive interference, referred to as the contrast sublayer (SCC), deposited on the surface of said transparent substrate; and
an absorbent layer referred to as sensitive layer (CS), distinct from said contrast sublayer and comprising between 1 and 5 sheets of at least one two-dimensional material.

2. The contrast-amplifying carrier as claimed in claim 1, wherein said at least one said two-dimensional material is a graphene-type material.

3. The contrast-amplifying carrier as claimed in one of the preceding claims, wherein said absorbent coating is discontinuous, forming a plurality of pads (P) on the surface of said substrate.

4. The contrast-amplifying carrier as claimed in either of claims 1 and 2, wherein said contrast sublayer is continuous and said sensitive layer is discontinuous, forming a plurality of pads (P') on the surface of said substrate.

5. The contrast-amplifying carrier as claimed in one of the preceding claims, wherein said sublayer is conductive and is linked by a conductive line (LC) to a contact pad (PC) allowing the application of an electrical potential.

6. The contrast-amplifying carrier as claimed in one of the preceding claims, wherein, at said illumination wavelength $\lambda$, the refractive index $n_0$ of the substrate, the real and imaginary parts of the complex refractive index of the contrast sublayer $N_1=n_1-jk_1$ and the thickness $e_1$ of the contrast sublayer meet the following conditions:

   •

$$\delta_1 = \frac{(n_0/n_3-1)}{2\nu_1\kappa_1}\left[1 - e^{-\frac{\kappa_1}{K}}\right];$$

   and
   • $k_1 \geq 0.001$, and preferably $k_1 \geq 0.01$, and more preferably still $k_1 \geq 0.1$
   • $k_1 < 0.15$; where:

   -

$$\delta_1 = \frac{2\pi n_0}{\lambda} e_1;$$

   -

$$\nu_1 = \frac{n_1}{\sqrt{n_0 n_3}};$$

   -

$$\kappa_1 = \frac{k_1}{\sqrt{n_0 n_3}};$$

   and
   -

$$K = \left\{[\pi/(n_0/n_3 - 1)]\sqrt{n_0/n_3}\right\}^{-1}$$

   with a tolerance lower than or equal to 0.2, preferably lower than or equal to 0.1, preferably lower than or equal to 0.02, and more preferably still lower than or equal to 0.01 for $\delta_1$.

7. The contrast-amplifying carrier as claimed in one of claims 1 to 5, wherein the refractive index of the substrate $n_0$, the complex refractive index of the contrast sublayer $N_1=n_1-jk_1$ and the thickness $e_1$ of the contrast sublayer further meet, with said tolerance and for said illumination wavelength $\lambda$, the following conditions:

   •

$$\delta_1 = \frac{n_0/n_3-1}{2\nu_1\kappa_1};$$

   and
   •

$$k_1 \geq 0.15$$

where:

-

$$\delta_1 = \frac{2\pi n_0}{\lambda} e_1;$$

-

$$v_1 = \frac{n_1}{\sqrt{n_0 n_3}};$$

-

$$\kappa_1 = \frac{k_1}{\sqrt{n_0 n_3}};$$

and

-

$$K = \left\{ [\pi/(n_0/n_3 - 1)]\sqrt{n_0/n_3} \right\}^{-1}$$

with a tolerance lower than or equal to 0.6, preferably lower than or equal to 0.3, preferably lower than or equal to 0.1, and more preferably still lower than or equal to 0.01 for $\delta_1$.

8. The contrast-amplifying carrier as claimed in either of claims 6 and 7, wherein, at said illumination wavelength $\lambda$, the refractive index $n_0$ of the substrate, the real and imaginary parts of the complex refractive index of the contrast sublayer $N_1 = n_1 - jk_1$ and the thickness $e_1$ of the contrast sublayer also meet the following condition:

$$v_1{}^2 = 1 + \kappa_1{}^2$$

with a tolerance lower than or equal to 20%, preferably lower than or equal to 5%, and more preferably still lower than or equal to 1% for $n_1$ and $k_1$.

9. The contrast-amplifying carrier as claimed in one of the preceding claims, wherein said antireflection absorbent coating exhibits, at normal incidence, a transmittance higher than or equal to 80%, preferably higher than or equal to 85% and more preferably still higher than or equal to 90% at said wavelength $\lambda$.

10. The contrast-amplifying carrier as claimed in one of the preceding claims, wherein said antireflection absorbent coating exhibits, at normal incidence, at said wavelength $\lambda$, a reflectivity lower than or equal to 1%, preferably lower than or equal to 0.5% and more preferably still lower than or equal to 0.1% and more preferably still lower than or equal to 0.05%.

11. The contrast-amplifying carrier as claimed in one of the preceding claims, wherein said sensitive layer is functionalized by the addition or the natural presence of ligands that are capable of binding at least one chemical or biological species.

12. A method for producing a contrast-amplifying carrier as claimed in one of the preceding claims, said method comprising a phase of designing said carrier and a phase of physically producing the carrier thus designed; **characterized in that** said design step comprises:

• selecting:

- a transparent material forming the substrate of said carrier;
- an absorbent material forming said absorbent sublayer;
- an ambient medium intended to come into contact with said coating on the side opposite said substrate; and
- a target wavelength $\lambda$;

• determining the thickness $e_1$ of said absorbent sublayer so as to meet the following condition:

$$\delta_1 = \frac{(n_0/n_3 - 1)}{2v_1\kappa_1}\left[1 - e^{-\frac{\kappa_1}{K}}\right]$$

with a tolerance lower than or equal to 0.1, preferably lower than or equal to 0.05, preferably lower than or equal to 0.01, and more preferably still lower than or equal to 0.005 for $\delta_1$; and

• selecting a two-dimensional material forming the sensitive layer of said carrier, suitable for being viewed with a negative contrast when it is deposited on said contrast sublayer, brought into contact with said ambient medium, illuminated through said substrate at said target wavelength $\lambda$ and also observed through said substrate. where:

- $n_0$ is the real refractive index of the substrate at the target wavelength $\lambda$;
- $N_1 = n_1\text{-}jk_1$, where $N_1 = n_1\text{-}jk_1$ is the complex refractive index of the contrast sublayer;
- $n_3 < n_0$ is the real refractive index of the ambient medium at the target wavelength $\lambda$;
-

$$\delta_1 = \frac{2\pi n_0}{\lambda} e_1;$$

-

$$v_1 = \frac{n_1}{\sqrt{n_0 n_3}};$$

-

$$\kappa_1 = \frac{k_1}{\sqrt{n_0 n_3}};$$

and

-

$$K = \left\{\left[\pi/\left(\frac{n_0}{n_3} - 1\right)\right]\sqrt{n_0/n_3}\right\}^{-1}.$$

**13.** A method for observing a sample including the following steps:

A. placing said sample (ECH) on the sensitive layer of a contrast-amplifying carrier as claimed in one of claims 1 to 11 and bringing it into contact with a transparent ambient medium (ME), the refractive index of which is lower than that of the substrate of said contrast-amplifying carrier;

B. illuminating said sample through said ambient medium, with an illumination cone including the normal incidence, with light radiation including at least one wavelength $\lambda$ such that the absorbent coating of said carrier behaves as an antireflection coating; and

C. observing the sample thus illuminated, also through said ambient medium.

14. A method for detecting or assaying at least one chemical or biological species or nanoparticles including the following steps:

I. providing a contrast-amplifying carrier as claimed in one of claims 1 to 11, comprising a functionalized sensitive layer (CF) that is capable of binding at least one chemical or biological species or nanoparticles;
II. bringing said functionalized surface or layer into contact with at least one solution (S) containing a chemical or biological species (ECD) that is capable of binding to said functionalized surface or layer, said solution being substantially transparent and exhibiting a refractive index that is lower than that of the substrate of said contrast-amplifying carrier;
III. illuminating said sample with an illumination cone including the normal incidence through said ambient medium at an illumination wavelength $\lambda$ such that the absorbent coating of said carrier behaves as an antireflection coating; and
IV. observing said contrast-amplifying carrier thus illuminated, also through said substrate.

15. The method as claimed in claim 14, wherein said step IV. is carried out by detecting fluorescence radiation or Raman scattering.

ME $\quad n_3$

CA $\quad N_1 = n_1 - jk_1 \qquad e_1$

MI

$n_0$

$\lambda \qquad$ FL

## FIG.1

FIG.2A

FIG.2B

FIG.2C

FIG.3

FIG.4A

FIG.4B

FIG.4C

FIG.4D

FIG.4E

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2015055809 A

- US 20150301039 A

**Littérature non-brevet citée dans la description**

- **AUSSERRÉ D ; ABOU KHACHFE R ; ROUSSILLE L ; BROTONS G ; VONNA L et al.** Anti-Reflecting Absorbing Layers for Electrochemical and Biophotonic Applications. *J Nanomed Nanotechnol,* 2014, vol. 5, 214
- **AUSSERRÉ D. ; ABOU KHACHFE R ; AMRA C. ; ZERRAD M.** *Absorbing Backside Anti-reflecting Layers for high contrast imaging in fluid cells.*
- **HOANG HIEP NGUYEN et al.** Surface Plasmon Resonance: A Versatile Technique for Biosensor Applications. *Sensors,* 2015, vol. 15, 10481-10510
- **HUA ZHANG.** Ultrathin Two-Dimensional Nanomaterials. *ACS Nano,* 2015, vol. 9 (10), 9451-9469
- **J-J. ADJIZIAN et al.** Dirac Cones in two-dimensional conjugated polymer networks. *Nat. Commun.,* 18 Décembre 2014, vol. 5, 5842

- Graphene Oxide Based Surface Plasmon Resonance Biosensors. **NAN-FU CHIU et al.** Advances in Graphene Science. InTech, 2013
- **J. AZEVEDO et al.** Versatile Wafer-Scale Technique for the Formation of Ultrasmooth and Thickness-Controlled Graphene Oxide Films Based on Very Large Flakes. *ACS Appl Mater Interfaces,* 30 Septembre 2015, vol. 7 (38), 21270-7
- **F. BONACCORSO et al.** Production and processing of graphene and 2d crystals. *Materials Today,* Décembre 2012, vol. 15 (12), 564-589
- **LUCIAN-GABRIEL ZAMFIR et al.** Synthesis and electroactivated addressing of ferrocenyl and azido-modified stem-loop oligonucleotides on an integrated electrochemical device. *Electrochimica Acta,* 2015, vol. 164, 62-70